# EUROPEAN PATENT APPLICATION

(11) **EP 3 831 367 A1**
(43) Date of publication of application: **09.06.2021**
(21) Application number: 19843277.5
(22) Date of filing: 01.08.2019
(51) Int. Cl.: A61K 9/20, A61J 3/10

(54) **THIN TABLET, METHOD FOR MANUFACTURING THIN TABLET, AND DEVICE FOR MANUFACTURING THIN TABLET**

(30) Priority: 03.08.2018 JP 2018147041
(71) Applicant: Daicel Corporation, Osaka 530-0011 (JP)
(72) Inventor: OKABAYASHI Tomohito, Tokyo 108-8230 (JP); HIRAMURA Takahiro, Tokyo 108-8230 (JP); HASHIKAWA Naohiro, Tokyo 108-8230 (JP)
(74) Representative: J A Kemp LLP
(86) International application number: PCT/JP2019/030146
(87) International publication number: WO 2020/027247

(57) **Abstract**

[Problem]

Provided are: a thin tablet that is easy to pick up, a thin tablet producing method, and a thin tablet producing apparatus.

[Solution]

A thin tablet 100 includes: a main body 110 including a planar surface 111; and protrusions 120 provided on the surface 111. When the thin tablet 100 is disposed on a virtual outer plane 130, a gap 135 is formed between at least a part of an outer edge 113 surrounding the surface 111 provided with the protrusion 120 and the outer plane 130 with the protrusion 120 in contact with the outer plane 130.

## Description

### Technical Field

The present invention relates to a thin tablet, a thin tablet producing method, and a thin tablet producing apparatus.

### Background Art

In the past, orally disintegrating tablets (for example, those disclosed in Patent Document 1) and easy-to-take solid preparations (for example, those disclosed in Patent Document 2) have been developed as highly convenient forms, which can safely be taken by patients who have difficulty in swallowing drugs, elderly people, children, etc. and which can easily be taken without water. For example, in the case of a specific patient who cannot recognize the necessity of taking a drug and spits out a tablet, a tablet that disintegrates in the oral cavity within 10 seconds (ultrafast disintegrating tablet) as disclosed in Patent Document 1 is required.

Ultrafast disintegrating tablets are formed, for example, in the form of thin tablets. Examples of thin tablets include straight tablets having a thin cylindrical shape, a diameter of about 14 mm or more, and a thickness of 0.5 mm or more and 1.5 mm or less. Thin tablets are used, for example, as ultrafast disintegrating tablets, but some of them are used for other applications.

### Citation List

### Patent Document

Patent Document 1: WO 2017/038455
Patent Document 2: WO 2017/002803

### Summary of Invention

### Technical Problem

When a thin tablet is taken out and dropped on a flat table, there is a problem that it is difficult for a person to pick it up with fingers because it is thin. In particular, when a medical personnel administers a thin tablet to a patient who cannot swallow the thin tablet by himself/herself, it is necessary to quickly pick up the thin tablet. Further, in the case of a thin tablet as an ultrafast disintegrating tablet, if it takes time to pick it up, the tablet may dissolve or be damaged in the hand or on the table.

One object of the present invention is to provide a thin tablet that is easy to pick up, a thin tablet producing method, and a thin tablet producing apparatus.

### Solution to Problem

More specifically, the present invention provides the following aspects.

### [First Aspect]

A thin tablet including:
a main body including a planar surface and
a protrusion provided on the surface,
wherein when the thin tablet is disposed on a virtual outer plane, a gap is formed between at least a part of an outer edge surrounding the surface, the surface being provided with the protrusion, and the outer plane, with the protrusion in contact with the outer plane.

### [Second Aspect]

The thin tablet according to the first aspect further including
a side surface extending from the outer edge of the surface, wherein an angle formed by the surface and the side surface is 90 degrees or less in the thin tablet.

### [Third Aspect]

The thin tablet according to the first or second aspect, wherein a thickness of the main body is 0.5 mm or more and 1.5 mm or less.

### [Fourth Aspect]

The thin tablet according to the first or second aspect, wherein a thickness of the main body is 0.5 mm or more and 1.2 mm or less.

### [Fifth Aspect]

The thin tablet according to any one of the first to fourth aspects, wherein a maximum width of the surface is 14 mm or more.

### [Sixth Aspect]

The thin tablet according to any one of the first to fifth aspects, wherein the protrusion occupies 90% or less of an area of the surface.

### [Seventh Aspect]

The thin tablet according to any one of the first to sixth aspects, wherein a height of the protrusion from the surface is 100% or less of a thickness of the main body.

### [Eighth Aspect]

The thin tablet according to any one of the first to seventh aspects, wherein the gap is 0.1 mm or more.

### [Ninth Aspect]

The thin tablet according to any one of the first to eighth aspects, wherein the protrusion is formed at a position overlapping a center of gravity of the main body in a normal direction of the surface.

### [Tenth Aspect]

The thin tablet according to any one of the first to eighth aspects, wherein the protrusion is formed at a position not overlapping a center of gravity of the main body in a normal direction of the surface.

### [Eleventh Aspect]

The thin tablet according to any one of the first to tenth aspects,
wherein the thin tablet includes: a first surface which is the surface and a second surface which is the surface,
the protrusion is provided on each of the first surface and the second surface,
the first surface and the second surface are parallel to each other, and
a side surface connects the outer edge of the first surface and the outer edge of the second surface in a normal direction of the first surface and the second surface.

### [Twelfth Aspect]

A method for producing the thin tablet described in any one of the first to eleventh aspects, wherein the thin tablet is produced by tableting.

### [Thirteenth Aspect]

The method for producing a thin tablet according to the twelfth aspect including:
preparing a plurality of molds that can be moved to a compression position and a separation position separated from each other,
charging a material into a space between the plurality of molds when the prepared plurality of molds are in the separation position,
moving to the compression position by applying force to the plurality of molds, and
tableting a thin tablet by compressing the charged material with the plurality of molds moved to the compression position,
wherein the plurality of molds are capable of forming a main body space for forming the main body of the thin tablet and a recess for forming the protrusion of the thin tablet at the compression position, and
the plurality of molds comprise: facing surfaces, which are planar surfaces for forming the surfaces of the main body, and the recess, which is recessed from the facing surface, at the compression position.

### [Fourteenth Aspect]

A thin tablet producing apparatus for producing the thin tablet described in any one of the first to eleventh aspects, wherein the thin tablet is produced by tableting.

### [Fifteenth Aspect]

The thin tablet producing apparatus according to the fourteenth aspect including:
a plurality of molds that can be moved to a compression position and a separation position separated from each other,
a charging path for charging a material into a space between the plurality of molds when the plurality of molds are in the separation position, and
a compression device that moves the plurality of molds to the compression position by applying a force and that tablets the thin tablet by compressing the charged material with the plurality of molds that have moved to the compression position,
wherein the plurality of molds are capable of forming a main body space for forming the main body of the thin tablet and a recess for forming the protrusion of the thin tablet at the compression position, and
the plurality of molds comprise: facing surfaces, which are planar surfaces for forming the surfaces of the main body, and the recess, which is recessed from the facing surface, at the compression position.

### Advantageous Effects of Invention

According to the present invention, it is possible to provide a thin tablet that is easy to pick up, a thin tablet producing method, and a thin tablet producing apparatus.

### Brief Description of Drawings

FIG. 1 is a perspective view of a thin tablet according to a first embodiment of the present invention.
FIG. 2 is a bottom view of the thin tablet illustrated in FIG. 1.
FIG. 3 is a front view of a thin tablet.
FIG. 4 is a front view of a thin tablet disposed on an outer plane.
FIG. 5 is a flowchart illustrating a thin tablet producing method of a thin tablet.
FIG. 6 is an exemplary diagram illustrating one step of a thin tablet producing method.
FIG. 7 is an exemplary diagram illustrating another step of the thin tablet producing method.
FIG. 8 is a partially enlarged view of a first mold and a second mold.
FIG. 9 is a front view of a thin tablet of a second embodiment.
FIG. 10 is a bottom view of a thin tablet of a third embodiment.
FIG. 11 is a bottom view of a thin tablet of a fourth embodiment.
FIG. 12 is a front view of a thin tablet of a fifth embodiment.
FIG. 13 is a bottom view of a thin tablet of a sixth embodiment.
FIG. 14 is a bottom view of a thin tablet of a seventh embodiment.
FIG. 15 is a front view of a thin tablet of a eighth embodiment.

### Description of Embodiments

The thin tablets of the first to eighth embodiments will be described below. In the first embodiment, the hundreds digit of each component is represented by 1. Similarly, each component of the second to eighth embodiments represents the hundreds digit by 2 to 8, respectively. Unless otherwise noted, components that differ only in the hundreds digit in different embodiments represent similar components.

In the present specification, an x direction, a y direction, and a z direction that are orthogonal to each other will be described. The x direction represents the opposite x1 direction and x2 direction. The y direction represents the opposite y1 direction and y2 direction. The z direction represents the opposite z1 direction and z2 direction. The z1 direction may be referred to as a downward direction, and the z2 direction may be referred to as an upward direction. Unless otherwise specified, these directions represent relative positional relationships and do not limit directions in actual use. The shape of the component is not limited to a strict geometric shape based on the described representation, as long as the technical ideas of the embodiments disclosed in the present specification are realized.

### (Whole of First Embodiment)

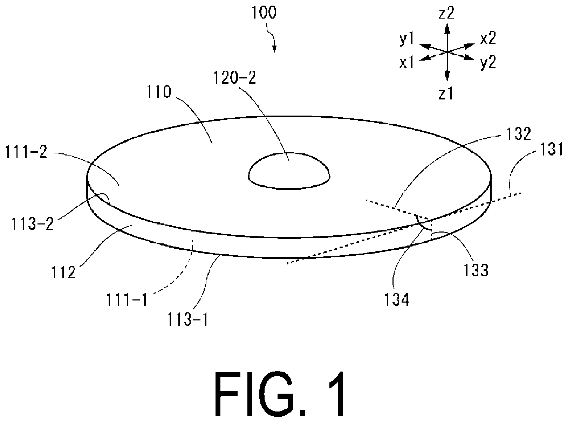

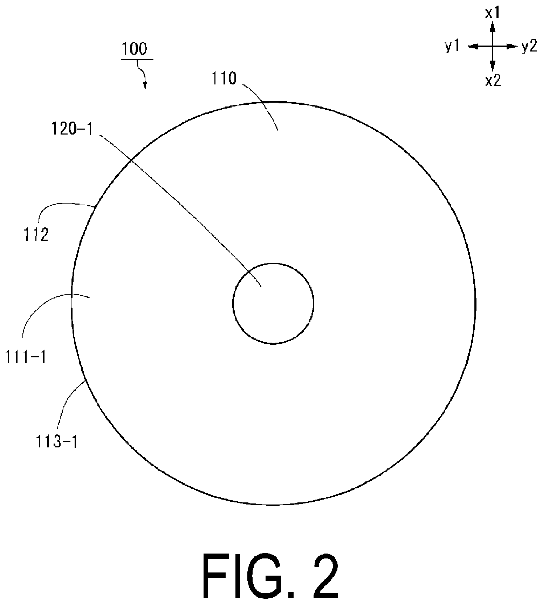

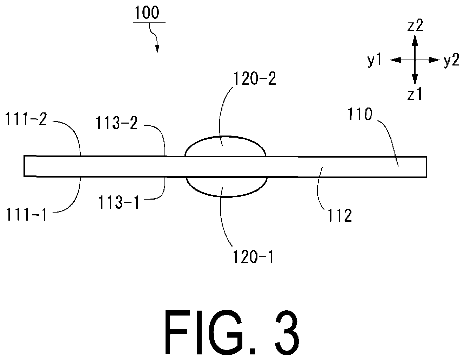

FIG. 1 is a perspective view of a thin tablet 100 of the first embodiment. FIG. 2 is a bottom view of the thin tablet 100. FIG. 3 is a front view of the thin tablet 100. As illustrated in FIG. 3, the thin tablet 100 includes a thin cylindrical main body 110 and further includes a first protrusion 120-1 and a second protrusion 120-2 both protruding from the main body 110 (hereinafter, may be referred to as a protrusion 120 without distinction).

### (Main body)

As illustrated in FIG. 1, the main body 110 is a thin cylinder having a central axis parallel to the z direction. As illustrated in FIG. 3, the main body 110 includes a first surface 111-1, a second surface 111-2, and a side surface 112. The first surface 111-1 and the second surface 111-2 (hereinafter, may be referred to as the surface 111 without distinction) have the same shape and have a shape translated from each other in the z direction. The first surface 111-1 is a circle oriented in the z1 direction and parallel to the xy plane (FIG. 2) and is surrounded by the first outer edge 113-1 (FIG. 2). The second surface 111-2 is a circle oriented in the z2 direction and parallel to the xy plane and is surrounded by the second outer edge 113-2. Hereinafter, the first outer edge 113-1 and the second outer edge 113-2 may be referred to as the outer edge 113 without distinction.

As illustrated in FIG. 1, the side surface 112 has a cylindrical shape that connects the first outer edge 113-1 and the second outer edge 113-2 in the normal direction (z direction) of the two surfaces 111. An angle 134 between the surface 111 and the side surface 112 is 90 degrees. The angle 134 between the surface 111 and the side surface 112 is the angle 134 formed by: a first virtual line 132 along the surface 111 and orthogonal to a virtual tangent line 131 of the outer edge 113; and a second virtual line 133 along the side surface 112 and orthogonal to the virtual tangent line 131 of the outer edge 113 in the thin tablet 100. In other words, in the rotationally symmetric main body 110, the angle 134 of the side surface 112 with respect to the surface 111 is expressed as the angle 134 formed by the surface 111 and the side surface 112 when cut in a plane passing through the center of rotation.

The thickness of the main body 110 of the present embodiment is 0.8 mm. In another example, the thickness of the main body 110 is, for example, 0.5 mm or more and 1.5 mm or less. In another example, the thickness of the main body 110 is, for example, 0.5 mm or more and 1.2 mm or less. The thickness of the main body 110 is defined to be parallel to the z direction orthogonal to the surface 111. In one example, the thin tablet 100 weighs 200 mg and is about 1.1 mm thick. In one example, the thin tablet 100 weighs 250 mg and is about 1.3 mm thick.

The maximum width of the main body 110 of the present embodiment, that is the diameter, is 14 mm. In another example, the maximum width of the surface 111 exceeds 14 mm. The width of the surface 111 is defined in a direction orthogonal to the thickness of the main body 110.

The main body 110 may use a tablet form called, for example, a straight tablet, a round-corner flat tablet, an angled-corner flat tablet, or the like.

### (Protrusion)

As illustrated in FIG. 3, the protrusions 120 are provided on each of the two surfaces 111. The first protrusion 120-1 projects from the first surface 111-1 in the z1 direction. The second protrusion 120-2 protrudes from the second surface 111-2 in the z2 direction. The position and shape of the second protrusion 120-2 with respect to the second surface 111-2 and the position and shape of the first protrusion 120-1 with respect to the first surface 111-1 are mirror-symmetric with respect to a virtual center plane parallel to the xy plane.

As illustrated in FIG. 1, the protrusion 120 has a convex smooth surface. As illustrated in FIG. 2, the protrusion 120 is a rotating body centered on an identical virtual axis to the main body 110. As a result, the thin tablet 100 is also a rotating body centered on the identical virtual axis to the main body 110 and the protrusion 120. The protrusion 120 and the main body 110 are integrally formed. The boundary between the protrusion 120 and the main body 110 is circular. When viewed from the z direction, the entire protrusion 120 does not protrude from the outer shape of the boundary between the protrusion 120 and the main body 110. The cross section of the protrusion 120 parallel to the xy plane becomes smaller as the distance from the surface 111 increases in the z direction.

As illustrated in FIG. 2, the proportion of the protrusion 120 to the area of the surface 111 is 5%. That is, the planar portion of the surface 111 is always exposed to the outside. The proportion of the protrusion 120 in the area of the surface 111 is preferably 90% or less. The height of the protrusion 120 from the surface 111 is preferably 100% or less of the thickness of the main body 110. In the present embodiment, the height of the protrusion 120 from the surface 111 is 100% of the thickness of the main body 110. The protrusion 120 of the present embodiment has a shape substantially obtained by cutting out a part of a sphere and is a small portion obtained by cutting the sphere at a ratio of 6:1 on a plane orthogonal to the diameter.

The protrusion 120 is formed at a position overlapping the center of gravity of the main body 110 in the normal direction or the z direction of the surface 111. That is, the center of the circle defining the boundary between the protrusion 120 and the surface 111 coincides with the center of the circle defining the surface 111.

### (Gap)

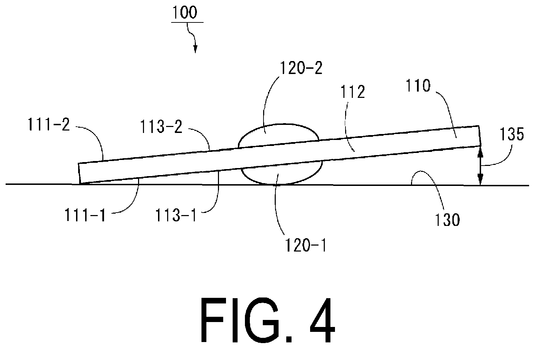

FIG. 4 is a front view in an exemplary state in which the thin tablet 100 is disposed on a virtual outer plane 130 that extends infinitely. The first surface 111-1 faces the outer plane 130. With the first protrusion 120-1 in contact with the outer plane 130, a gap 135 of 0.1 mm or more is formed between the outer plane 130 and at least a part of the first outer edge 113-1 surrounding the first surface 111-1 provided with the first protrusion 120-1. The protrusion 120 is preferably formed in which the gap 135 is 0.1 mm or more. When the thin tablet 100 is disposed on the virtual outer plane 130 under gravity, the protrusion 120 is preferably formed in which the gap 135 of 0.1 mm or more is formed without applying an outer force.

FIG. 4 illustrates a state in which the left side of the first outer edge 113-1 is in contact with the outer plane 130. Since the first outer edge 113-1 is circular and the protrusion 120 overlaps the center of gravity of the main body 110 in the z direction of FIG. 3, which part of the first outer edge 113-1 comes into contact with the outer plane 130 is not limited under gravity. The place where the thin tablet 100 is actually disposed is not limited to a perfect plane.

### (Thin tablet producing method)

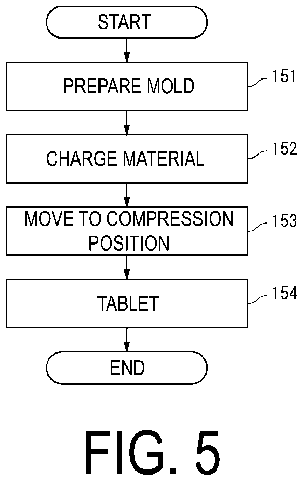

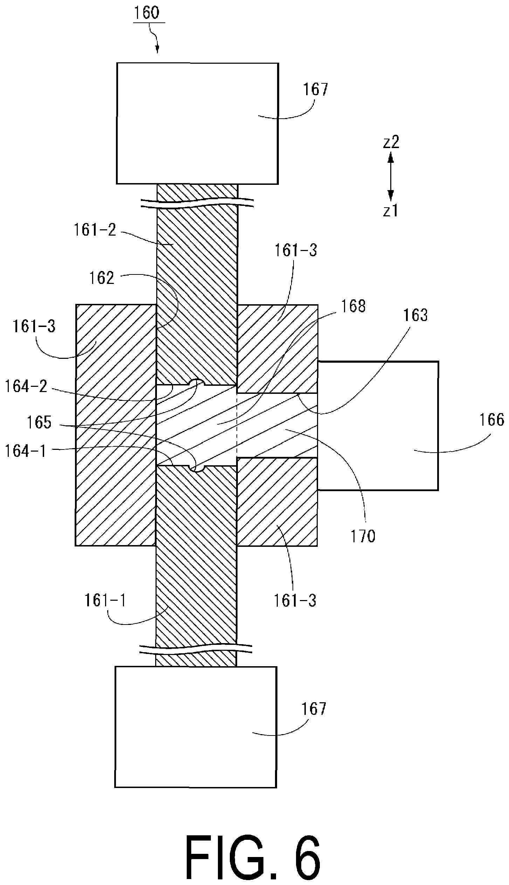

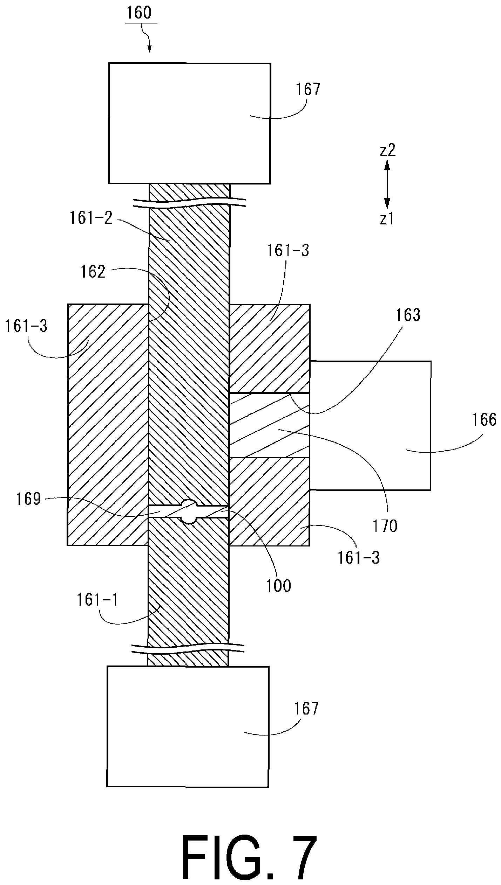

FIG. 5 is a flowchart illustrating a thin tablet producing method for the thin tablet 100. FIGS. 6 and 7 are exemplary views illustrating a portion of the process of the thin tablet producing method, including a partial cross section of a thin tablet producing apparatus 160.

As illustrated in FIG. 6, the thin tablet producing apparatus 160 includes a first mold 161-1, a second mold 161-2, and a third mold 161-3 (hereinafter, they may be referred to as a plurality of molds 161 or simply molds 161 without distinction).

The third mold 161-3 includes a columnar vertical space 162 penetrating the third mold 161-3 in the z direction. The third mold 161-3 includes an charging path 163 penetrating in the direction intersecting the z direction from the middle of the vertical space 162 in the z direction to the outside of the third mold 161-3.

Each of the first mold 161-1 and the second mold 161-2 is a cylinder extending in the z direction. The first mold 161-1 is located on the z1 side of the second mold 161-2. Within the cross section parallel to the xy plane, the radii of the first mold 161-1 and the second mold 161-2 are slightly smaller than the radius of the vertical space 162. The vicinity of the z2 side end of the first mold 161-1 and the vicinity of the z1 side end of the second mold 161-2 can move in the z direction in the vertical space 162 while being in close contact with the vertical space 162.

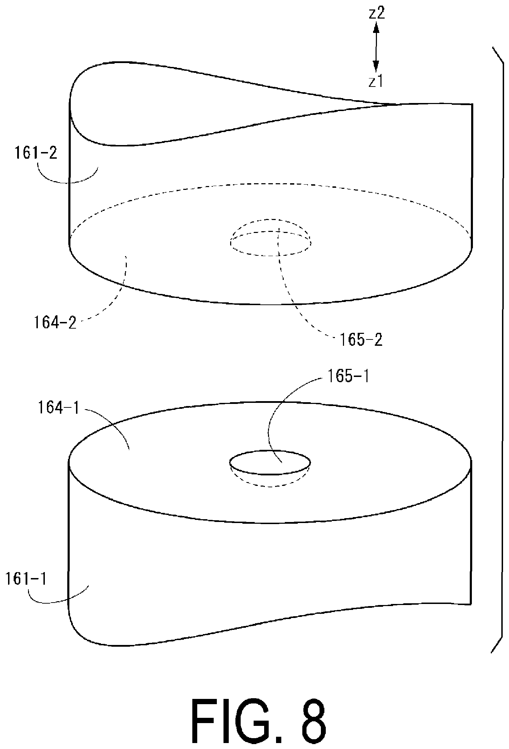

FIG. 8 is a partially enlarged view of the first mold 161-1 and the second mold 161-2. The first mold 161-1 includes a first facing surface 164-1 facing the z2 direction at the end on the z2 side. The second mold 161-2 includes a second facing surface 164-2 facing the z1 direction at the end on the z1 side. The first facing surface 164-1 and the first facing surface 164-1 are parallel to the xy plane and are disposed to face each other. The first mold 161-1 includes a first recess 165-1 recessed in the z1 direction in the center of the first facing surface 164-1. The second mold 161-2 includes a second recess 165-2 recessed in the z2 direction in the center of the second facing surface 164-2.

The first facing surface 164-1 (FIG. 8) is a planar surface for forming the first surface 111-1 (FIG. 3) and has substantially the same shape as the first surface 111-1. The first recess 165-1 (FIG. 8) is a portion for forming the first protrusion 120-1 (FIG. 3) and has a shape complementary to the first protrusion 120-1. That is, the space defined in the first recess 165-1 is substantially the same as the shape of the first protrusion 120-1.

In the state of FIG. 6, since the first mold 161-1 and the second mold 161-2 are separated from each other, it is expressed that the mold 161 is in a separation position. In the state of FIG. 7, since the first mold 161-1 and the second mold 161-2 are closer than in the case of the separation position, it is expressed that the mold 161 is in a compression position. The plurality of molds 161 can be moved to a compression position and a separation position.

As illustrated in FIG. 6, the thin tablet producing apparatus 160 further includes a charging device 166 and a compression device 167. The charging device 166 charges a material 170 of the thin tablet 100 into the vertical space 162 through the charging path 163. The charging device 166 includes, for example, a tank for storing the material 170 and a pump for transporting the material 170 from the tank.

The compression device 167 can move the first mold 161-1 and the second mold 161-2 relative to the third mold 161-3. Further, the compression device 167 can move the first mold 161-1 and the second mold 161-2 relative to each other. In particular, when the compression device 167 moves the first mold 161-1 and the second mold 161-2 from the separation position toward the compression position, a strong compressive force is applied to bring the first mold 161-1 and the second mold 161-2 close to each other.

The compression device 167 is, for example, an eccentric roller, a device that applies a force to the mold 161 by hydraulic pressure, or a device that moves the mold 161 by a motor and a gear. As the thin tablet producing apparatus 160, a related-art tableting apparatus may be used except for the mold 161.

In the thin tablet producing method, first, as illustrated in step 151 (FIG. 5), the plurality of molds 161 (FIG. 6) that can be moved to a separation position that is separated from each other (FIG. 6) and a compression position (FIG. 7) are prepared.

Next, in step 152 (FIG. 5), as illustrated in FIG. 6, when the prepared plurality of molds 161 are in the separation positions, the charging device 166 charges the material 170 into a space 168 between the plurality of molds 161 via the charging path 163. The space 168 between the plurality of molds 161 is sandwiched between the first mold 161-1 and the second mold 161-2 in the z direction, partially surrounded by the third mold 161-3 in a direction orthogonal to the z direction, and open to the charging path 163.

Next, in step 153 (FIG. 5), as illustrated in FIG. 7, the compression device 167 moves the plurality of molds 161 to the compression position by applying a force to the plurality of molds 161. The compression device 167 shifts the first mold 161-1 and the second mold 161-2 from the charging path 163 in the z1 direction before compression. The plurality of molds 161 form: a main body space 169 for forming the main body 110 of the thin tablet 100; and the recess 165 (FIG. 8) for forming the protrusion 120 of the thin tablet 100 at the compression position.

The main body space 169 formed at the compression position is substantially equal to the shape of the main body 110 of the thin tablet 100. The main body space 169 is sandwiched between the first facing surface 164-1 (FIG. 8) and the second facing surface 164-2 (FIG. 8) from the z direction. The main body space 169 is surrounded by a cylindrical inner surface that defines the vertical space 162 from a direction orthogonal to the z direction. The inner surface that defines the vertical space 162 becomes the mold of the side surface 112 of the main body 110.

Next, in step 154 (FIG. 5), as a result of the compression in step 153 (FIG. 5), the compression device 167 illustrated in FIG. 7 tablets the thin tablet 100 by compressing the material 170 (FIG. 6) charged by the plurality of molds 161 that have moved to the compression position.

In another example, the plurality of molds may be composed of three or more, or may be two or less. In another example, the main body space 169 may be formed in one mold or three or more molds, and the protrusion 120 may be formed in two or more molds. The plurality of molds may have other configurations capable of forming the main body space 169 and the recess 165 corresponding to the thin tablet 100 at the compression position.

In another example, the thin tablet 100 may be produced by first forming the main body 110 by, for example, tableting, and then adding the protrusion 120 to the main body 110. In another example, the main body 110 and the protrusions 120 may be formed by scraping a solid larger than the thin tablet 100. The thin tablet producing apparatus 160 is, for example, a rotary tableting machine, a single punch tableting machine, and a hand press machine.

### (Orally disintegrating tablet)

The thin tablet 100 is, for example, an orally disintegrating tablet. The disintegration time of the thin tablet 100 in water is about 7 seconds or less, preferably 5 seconds or less in one example. The oral disintegration time of the thin tablet 100 is 6 seconds or less, preferably 5 seconds or less in one example.

The medicinal ingredient contained in the thin tablet 100 is a pharmaceutical ingredient and a nutritional ingredient in foods and health foods. As the medicinal ingredient, the medicinal ingredient alone or the medicinal ingredient coated or granulated for the purpose of sustained release or bitterness masking may be added. There are no particular restrictions on the application, type, etc. of the medicinal ingredient contained in the thin tablet 100.

As necessary, the thin tablet 100 may contain, in addition to the medicinal ingredient, any other optional pharmaceutically acceptable ingredients such as excipients, surfactants, lubricants, acidulants, sweeteners, flavoring agents, spices, colorants, and stabilizers. As these optional ingredients, for example, the ingredients described in the Japanese Pharmaceutical Excipients Dictionary (Yakuji Nippo, Ltd.) and the Japanese Pharmacopoeia can be used. Further, as long as the desired effect of the present invention is exhibited, the blending ratio of each component is not particularly limited and can be appropriately determined by those skilled in the art.

The material 170 (FIG. 6) of the thin tablet 100 is a mixture obtained by mixing a disintegrating particle composition with a medicinal ingredient (or a pharmaceutical composition containing the medicinal ingredient) and other optional ingredients described above. The thin tablet producing apparatus 160 described above is a suitable tableting machine known to those skilled in the art, except for the shape of the mold 161. The thin tablet 100 is tableted with, for example, a tableting compressive force of about 2 to 20 kN, preferably about 5 to 20 kN. It is also possible to use a method called "external lubrication tableting method" in which a lubricant such as magnesium stearate is sprayed or applied to the mold 161 of a tableting machine (also called a mortar or pestle) in advance to smooth the surface.

The disintegrating particle composition contains, for example, acid-type carboxymethyl cellulose as a disintegrant component. Various optional components known to those skilled in the art may be appropriately added to and mixed with the disintegrating particle composition, for example, for the purpose of adjusting various properties such as disintegrating force, binding force, and feeling of taking tablets. Examples of such components include fluidizers, sweeteners, spices, colorants, and the like.

A person skilled in the art can appropriately determine the blending amount of each component in the disintegrating particle composition according to: the type of each component, the type and application of the medicinal ingredient to be used in the disintegrating particle composition, the application of the orally disintegrating tablet as the final product, and the like.

### (Easy-to-take solid preparation)

The thin tablet 100 is an easy-to-take solid preparation in addition to being an orally disintegrating tablet or in place of an orally disintegrating tablet. "Easy to take" generally means that a solid preparation or the like has the property and characteristics of being easy to drink (easy to swallow). In one example, the thin tablet 100 contains a gelling agent that is slippery when in contact with water.

### (Modification)

In another example, the angle between the surface and the side surface is less than 90 degrees. In such another example, for example, the second surface is concentric with the first surface, and the second surface is a circle with a smaller radius than the first surface. That is, it is a truncated cone.

### (Conclusion)

According to the present embodiment, since the protrusion 120 is provided on the surface 111, when the thin tablet 100 is placed on the outer plane 130 (for example, the floor), the gap 135 is formed between the outer edge 113 of the surface 111 and the outer plane 130, making it easy to pick up the thin tablet 100.

According to the present embodiment, when the angle between the surface 111 and the side surface 112 is 90 degrees or less, it is particularly difficult to pick up the thin tablet 100 without the protrusion 120, but since the protrusion 120 is provided, the gap 135 is formed between the outer edge 113 of the surface 111 and the outer plane 130, making it easy to pick up the thin tablet 100.

According to the present embodiment, in the thin tablet 100 which is difficult to pinch because the thickness of the main body 110 is 0.5 mm or more and 1.5 mm or less, or 0.5 mm or more and 1.2 mm or less, the gap 135 is formed between the surface 111 and the outer plane 130 to facilitate the picking up of the thin tablet 100.

According to the present embodiment, in the thin tablet 100 which is difficult to pinch because the maximum width of the surface 111 is 14 mm or more, the gap 135 is formed between the surface 111 and the outer plane 130, thus the thin tablet 100 can be easily picked up.

According to the present embodiment, since the proportion of the protrusion 120 to the area of the surface 111 is 90% or less, it is possible to make it easier to pick up the thin tablet 100 while preventing the thin tablet 100 from becoming thicker than necessary due to the protrusion 120.

According to the present embodiment, since the height of the protrusion 120 from the surface 111 is 100% or less of the thickness of the main body 110, it is possible to make it easier to pick up the thin tablet 100 while preventing the thin tablet 100 from becoming thicker than necessary due to the protrusion 120.

According to the present embodiment, since the gap 135 of 0.1 mm or more is formed between at least a part of the outer edge 113 and the outer plane 130, it is easy to pick up the thin tablet 100 with a human finger.

According to the present embodiment, since the protrusion 120 overlaps the center of gravity of the main body 110, it is easy to tilt the main body 110 in various directions around the protrusion 120.

According to the present embodiment, since each of the two surfaces 111 has the protrusion 120, even when any of the surfaces 111 faces the outer plane 130, the gap 135 is formed between the surface 111 and the outer plane 130, making it easy to pick up the thin tablet 100.

According to the present embodiment, when the thin tablet 100 is at least one of the orally disintegrating tablet or the easy-to-take solid preparation, the gap 135 makes it possible to pick up the tablet quickly, and thus it is easy to prevent the tablet from collapsing and gelling when picking up the tablet.

### (Second Embodiment)

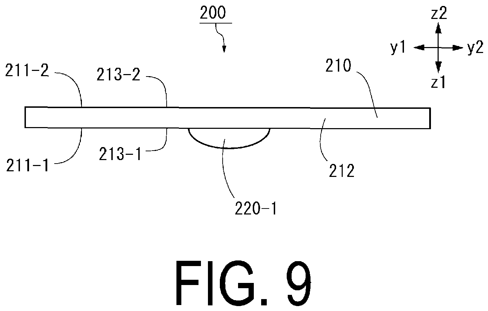

FIG. 9 is a front view of a thin tablet 200 of the second embodiment. The thin tablet 200 does not include the second protrusion 120-2 of the thin tablet 100 of the first embodiment illustrated in FIG. 3. A second surface 211-2 illustrated in FIG. 9 is a plane. In another example, the second surface 211-2 may be non-planar.

According to the present embodiment, since a first protrusion 220-1 is provided on a first surface 211-1, when the thin tablet 200 is placed on an outer plane (for example, a floor), a gap is formed between an outer edge 213 of the surface 211 and the outer plane, making it easier to pick up the thin tablet 200.

### (Third Embodiment)

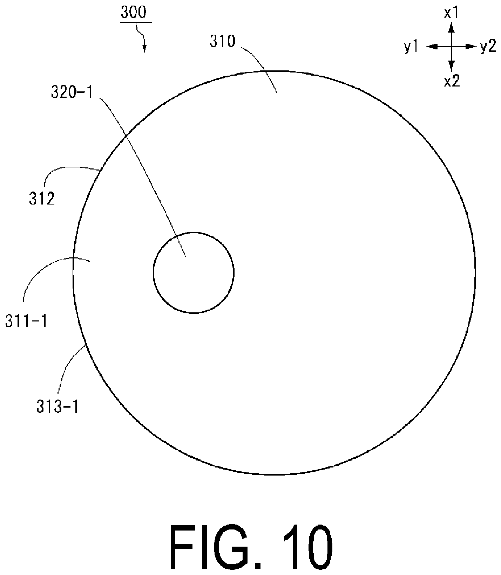

FIG. 10 is a bottom view of a thin tablet 300 of the third embodiment. The position of a first protrusion 320-1 of the present embodiment is different from that of the first protrusion 120-1 (FIG. 2) of the first embodiment. The first protrusion 320-1 of the present embodiment is deviated from the center of a first surface 311-1 in the y1 direction. The first protrusion 320-1 does not overlap the center of gravity of a main body 310 in the z direction.

According to the present embodiment, since the first protrusion 320-1 does not overlap the center of gravity of the main body 310, the main body 310 does not rotate around the first protrusion 320-1, and the main body 310 is easy to stabilize.

### (Fourth Embodiment)

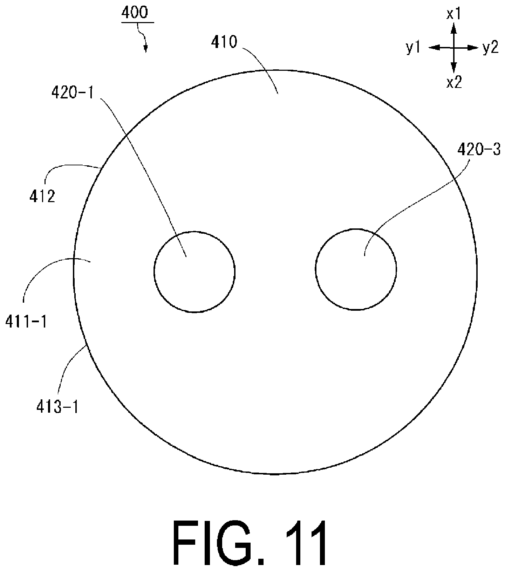

FIG. 11 is a bottom view of a thin tablet 400 of the fourth embodiment. The thin tablet 400 of the present embodiment includes a first protrusion 420-1 and a third protrusion 420-3 protruding from a first surface 411-1. The third protrusion 420-3 has the same shape as the first protrusion 420-1 and has a shape that is translated. The positions of the first protrusion 420-1 and the third protrusion 420-3 may or may not overlap the center of gravity of a main body 410 in the z direction. The shape of the first protrusion 420-1 and the shape of the third protrusion 420-3 may be the same or different. In addition to the third protrusion 420-3, one or more protrusions may be similarly provided.

According to the present embodiment, since the first protrusion 420-1 and the third protrusion 420-3 are provided on the first surface 411-1, the thin tablet 400 can be placed more stably than in the case where only the first protrusion 420-1 is provided. A plurality of protrusions including the first protrusion 420-1 and the third protrusion 420-3 may be disposed not to stabilize the thin tablet 400.

### (Fifth Embodiment)

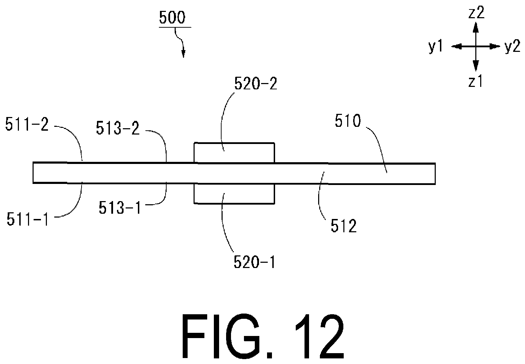

FIG. 12 is a front view of a thin tablet 500 of the fifth embodiment. A first protrusion 520-1 and a second protrusion 520-2 of the present embodiment are cylindrical with a central axis parallel to the z direction.

According to the present embodiment, since the tip of the protrusion 520 far from a main body 510 is a plane, a gap can be formed between an entire outer edge 513 and the virtual outer plane 130 (FIG. 4). By providing a plurality of the protrusions 520, or by increasing the contact area of the protrusions 520 with respect to the virtual outer plane 130 (FIG. 4), a gap may be formed between the entire outer edge 513 and the virtual outer plane 130 (FIG. 4). In another example, by making the contact area between the protrusion 520 and the outer plane 130 larger than that of the first embodiment, a gap may be formed between the entire outer edge 513 and the virtual outer plane 130 (FIG. 4). For example, the protrusion 520 may be disposed to draw characters when viewed from the z direction.

### (Sixth Embodiment)

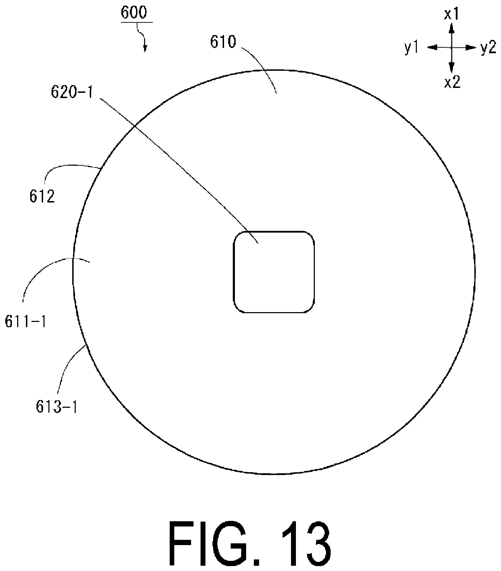

FIG. 13 is a bottom view of a thin tablet 600 of the sixth embodiment. A first protrusion 620-1 of the present embodiment is a rounded quadrangular pyramid. The outer shape of the first protrusion 620-1 is a square with rounded corners when viewed in the z direction. That is, the contact portion between a first surface 611-1 and the first protrusion 620-1 is a square with rounded corners. The cross section of the first protrusion 620-1 parallel to the xy plane becomes smaller as it moves away from the first surface 611-1.

As in the present embodiment, the outer shapes of a first outer edge 613-1 of the main body 610 and the first protrusion 620-1 may be different when viewed in the z direction. In another example, the outer shape of the first protrusion 620-1 may be another polygon with rounded corners. In another example, the outer shape of the first protrusion 620-1 may be a polygon with sharp corners. In another example, the first protrusion 620-1 may be a pillar obtained by moving a polygon with rounded corners or a polygon with sharp corners in the z direction. In another example, the outer shape of the protrusion 620 seen from the z direction may have a complicated shape such as a flower shape or a fish shape.

### (Seventh Embodiment)

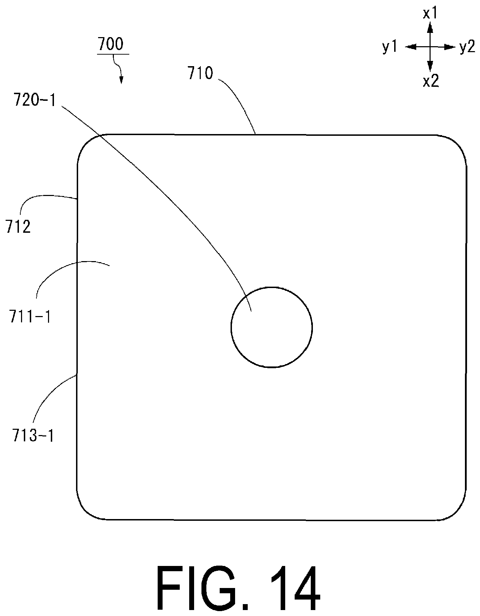

FIG. 14 is a bottom view of a thin tablet 700 of the seventh embodiment. A first outer edge 713-1 of a first surface 711-1 of the present embodiment is a square with rounded corners. A main body 710 is a pillar obtained by a locus of movement on the first surface 711-1 in the z direction.

As in the present embodiment, the outer shapes of the first outer edge 713-1 of the main body 710 and a first protrusion 720-1 may be different when viewed in the z direction. In another example, the first outer edge 713-1 may be another polygon with rounded corners. In another example, the first outer edge 713-1 may be a polygon with sharp corners. In another example, the first outer edge 713-1 may have other shape. In another example, the shape of the surface 711 seen from the z direction may be a complicated shape such as a flower shape or a fish shape.

### (Eighth Embodiment)

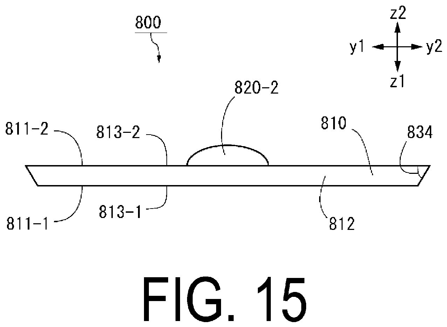

FIG. 15 is a front view of a thin tablet 800 of the eighth embodiment. The thin tablet 800 does not include the first protrusion 120-1 of the thin tablet 100 of the first embodiment illustrated in FIG. 3. An angle 834 between a second surface 811-2 and a side surface 812 illustrated in FIG. 15 is about 60 degrees. That is, a main body 810 has a truncated corn shape, and the cross section parallel to the xy plane becomes smaller from the second surface 811-2 toward a first surface 811-1. The first surface 811-1 and the second surface 811-2 illustrated in FIG. 15 are planar. In another example, the first surface 811-2 may be non-planar.

When the thin tablet 100 is disposed with the second surface 811-2 facing the outer plane, since an angle 834 between the second surface 811-2 and the side surface 812 is less than 90 degrees, it is more difficult to pick up the tablet than when the angle 834 is 90 degrees or more. According to the present embodiment, a second protrusion 820-2 creates a gap between an outer edge 813 of the second surface 811-2 and the outer plane, making it easier to pick up the thin tablet 200.

The above embodiments and modifications may be combined as long as the technical ideas disclosed in the present specification are realized.

### Reference Signs List

- 100: Thin tablet
- 110: Main body
- 111: Surface
- 112: Side surface
- 113: Outer edge
- 120: Protrusion
- 130: Outer plane
- 131: Virtual tangent line
- 132: First virtual line
- 133: Second virtual line
- 134: Angle
- 135: Gap
- 160: Thin tablet producing apparatus
- 161: Mold
- 162: Vertical space
- 163: Charging path
- 164-1: First facing surface
- 164-2: Second facing surface
- 165: Recess
- 166: Charging device
- 167: Compression device
- 168: Space
- 169: Main body space
- 170: Material

## Claims

1. A thin tablet comprising:
a main body comprising a surface that is planar and
a protrusion provided on the surface,
wherein when the thin tablet is disposed on an outer plane that is virtual, a gap is formed between at least a part of an outer edge surrounding the surface, the surface being provided with the protrusion, and the outer plane, with the protrusion in contact with the outer plane.

2. The thin tablet according to claim 1 further comprising
a side surface extending from the outer edge of the surface, wherein an angle formed by the surface and the side surface is 90 degrees or less in the thin tablet.

3. The thin tablet according to claim 1 or 2, wherein a thickness of the main body is 0.5 mm or more and 1.5 mm or less.

4. The thin tablet according to claim 1 or 2, wherein a thickness of the main body is 0.5 mm or more and 1.2 mm or less.

5. The thin tablet according to any one of claims 1 to 4, wherein a maximum width of the surface is 14 mm or more.

6. The thin tablet according to any one of claims 1 to 5, wherein the protrusion occupies 90% or less of an area of the surface.

7. The thin tablet according to any one of claims 1 to 6, wherein a height of the protrusion from the surface is 100% or less of a thickness of the main body.

8. The thin tablet according to any one of claims 1 to 7, wherein the gap is 0.1 mm or more.

9. The thin tablet according to any one of claims 1 to 8, wherein the protrusion is formed at a position overlapping a center of gravity of the main body in a normal direction of the surface.

10. The thin tablet according to any one of claims 1 to 8, wherein the protrusion is formed at a position not overlapping a center of gravity of the main body in a normal direction of the surface.

11. The thin tablet according to any one of claims 1 to 10,
wherein the thin tablet comprises: a first surface which is the surface and a second surface which is the surface,
the protrusion is provided on each of the first surface and the second surface,
the first surface and the second surface are parallel to each other, and
a side surface connects the outer edge of the first surface and the outer edge of the second surface in a normal direction of the first surface and the second surface.

12. A method for producing the thin tablet described in any one of claims 1 to 11, wherein the thin tablet is produced by tableting.

13. The method for producing a thin tablet according to claim 12 comprising:
preparing a plurality of molds that can be moved to a compression position and a separation position separated from each other,
charging a material into a space between the plurality of molds when the plurality of molds that are prepared are in the separation position,
moving to the compression position by applying force to the plurality of molds, and
tableting a thin tablet by compressing the material, which is charged, with the plurality of molds moved to the compression position,
wherein the plurality of molds are capable of forming a main body space for forming the main body of the thin tablet and a recess for forming the protrusion of the thin tablet at the compression position, and
the plurality of molds comprise: facing surfaces, which are planar surfaces for forming the surfaces of the main body, and the recess, which is recessed from the facing surface, at the compression position.

14. A thin tablet producing apparatus for producing the thin tablet described in any one of claims 1 to 11, wherein the thin tablet is produced by tableting.

15. The thin tablet producing apparatus according to claim 14 comprising:
a plurality of molds that can be moved to a compression position and a separation position separated from each other,
a charging path for charging a material into a space between the plurality of molds when the plurality of molds are in the separation position, and
a compression device that moves the plurality of molds to the compression position by applying a force and that tablets the thin tablet by compressing the material, which is charged, with the plurality of molds that have moved to the compression position,
wherein the plurality of molds are capable of forming a main body space for forming the main body of the thin tablet and a recess for forming the protrusion of the thin tablet at the compression position, and
the plurality of molds comprise: facing surfaces, which are planar surfaces for forming the surfaces of the main body, and the recess, which is recessed from the facing surface, at the compression position.
